Europäisches Patentamt

European Patent Office

Office européen des brevets

(19)

(11) Publication number: **0 079 611**
**A2**

(12) **EUROPEAN PATENT APPLICATION**

(21) Application number: **82110583.0**

(22) Date of filing: **16.11.82**

(51) Int. Cl.³: **A 61 K 7/18**
**A 61 K 7/22**

(30) Priority: **18.11.81 US 322624**

(43) Date of publication of application:
**25.05.83 Bulletin 83/21**

(84) Designated Contracting States:
**AT BE CH DE FR GB IT LI LU NL SE**

(71) Applicant: **Richardson-Vicks, Inc.**
**Ten Westport Road**
**Wilton, Conn. 06897(US)**

(72) Inventor: **Shah, Nutan B.**
**160 Berrian Road**
**New Rochelle New York 10804(US)**

(74) Representative: **Vossius Vossius Tauchner Heunemann Rauh**
**Siebertstrasse 4 P.O. Box 86 07 67**
**D-8000 München 86(DE)**

(54) Oral compositions containing strontium EDTA complex and soluble fluoride.

(57) Fluoride containing oral compositions having strontium EDTA Complex present as a stain and tartar removing agent.

EP 0 079 611 A2

VOSSIUS · VOSSIUS · TAUCHNER · HEUNEMANN · RAUH

PATENTANWÄLTE

0079611

SIEBERTSTRASSE 4 · 8000 MÜNCHEN 86 · PHONE: (089) 47 40 75
CABLE: BENZOLPATENT MÜNCHEN · TELEX 5-29 453 VOPAT D

Our ref.: S 117 EP
Case    : V-1188

Richardson-Vicks Inc.

Wilton, Conn., USA                    November 16, 1982

ORAL COMPOSITIONS CONTAINING

STRONTIUM EDTA COMPLEX AND SOLUBLE FLUORIDE

Field of the Invention

This invention relates to oral compositions and more particularly to dentifrices containing suitable available soluble fluoride and is particularly concerned with fluoride-containing dentifrices having strontium EDTA complex present as a stain and tartar removing agent.

Background of the Invention

It has long been known that treatment of tooth surfaces with fluorides has a caries-inhibiting effect. To obtain this effect, fluoride compounds have been incorporated into dentifrices.

More recently, it has also been known that incorporation of a strontium chelate with ethylenediaminetetraacetate acid (EDTA) into a dentifrice is of great benefit for the treatment of hypersensitive dentin, gingivitis and periodontitis - see for example U.S. Patents 3,699,221 issued October 17, 1972 and 3,988,434 issued October 26, 1976 both to
M. Schole et al.

While it might appear desirable to produce a
dentifrice having the benefits of both the strontium
EDTA complex and fluoride, this has not heretofore
been possible due to inactivation of the soluble
fluoride by strontium.  That is, a dentifrice
containing both a soluble fluoride and strontium
ion loses an appreciable amount of available
fluoride from the composition upon aging.  It
has been found that the soluble fluoride is removed
from the composition by forming insoluble and inac-
tive strontium fluoride, $SrF_2$, thereby reducing the
anticariogenic effectiveness of the fluoride
dentifrice.

This is important because the U.S. Food and Drug
Administration has considered that a dentifrice
composition made with sodium monofluorophosphate
must show more than 600 ppm available fluoride in
an aged product for it to be effective as an anticaries
product.

It is significant to note that none of the prior
references to the use of strontium EDTA complexes
in dentifrices discloses or suggests that soluble
fluoride compounds could be incorporated into dentifrices containing said strontium complex.  Recognition of the problem of inactivation of soluble
fluoride by the formation of insoluble and inactive strontium fluoride has precluded such a disclosure.

Moreover, when it was attempted to put such caries-inhibiting fluoride compounds into the dentifrice compositions disclosed in the aforementioned Schole et al patents inactivation of the soluble fluoride did in fact occur as expected. Additionally, even when the compositions disclosed in the Schole et al patents were modified so as to remove other clearly interferring substances, such as the water-insoluble barium and strontium salt abrasives employed by Schole et al, the fluoride was still unacceptably inactivated by the strontium EDTA complex formed in accordance with the teachings and disclosure of the Schole et al patents. It was, therefore, heretofore not considered possible to incorporate soluble fluoride anti-caries compound into dentifrice compositions containing strontium EDTA complex.

It is, therefore, an object of this invention to provide oral compositions and more particularly dentifrice compositions containing both a source of soluble fluoride and strontium EDTA complex with improved levels of soluble fluoride availability maintained over a prolonged period of time.

## Summary of the Invention

It has now been surprisingly found that it is possible to obtain oral compositions and more particularly dentifrice compositions containing both a source of soluble fluoride and strontium EDTA complex with
acceptable levels of soluble fluoride availability
maintained over a long period of time when one
employs as the strontium EDTA complex, strontium EDTA
complex substantially free of strontium ions from
sources other than the strontium EDTA complex. That
is, it has been unexpectedly discovered that the
inactivation of soluble fluoride by the strontium
EDTA complex was not in fact due to the strontium
EDTA complex itself but to strontium ions present
in the product as a result of by-products formed
in the production of the strontium EDTA complex.
Thus, it has been unexpectedly discovered that if
one produces the strontium EDTA complex by processes
wherein the strontium EDTA complex formed is substantially free of strontium ion from sources other than
the strontium EDTA complex it is then possible to
add soluble fluoride compounds to the oral compositions without unacceptable inactivation of the
fluoride.

## Details of the Invention

It has been discovered that the presence of strontium ions from sources other than strontium EDTA complex itself has previously prevented the acceptable
incorporation of soluble fluoride compounds into
oral compositions and in particular, in dentifrice
compositions, when said compositions contained strontium EDTA complex. Therefore, if one produces strontium EDTA complex by a process wherein the strontium EDTA complex so produced is substantially free of strontium ion from sources other than the strontium EDTA complex then one can add soluble fluoride compounds to the compositions without unacceptable loss of available fluoride.

Strontium EDTA complex substantially free of strontium ion from sources other than the strontium EDTA complex can be produced for example, by the process disclosed in U.S. Patent 4,224,310 issued September 23, 1980 to N. Shah and assigned to Richardson-Merrell Inc., which patent is incorporated herein by reference. Alternatively, employing the process described in said patent but employing the appropriate molar amount of strontium hydroxide similarly produces a strontium EDTA complex substantially free of strontium ion from sources other than the strontium EDTA complex. One cannot employ a strontium EDTA complex containing strontium ions from sources other than the strontium EDTA complex such as is obtained when the complex is formed according to the two aforementioned Schole et al U.S. patents where the complex is formed from strontium chloride and the disodium salt of ethylenediaminetetraacetic acid.

Thus, this invention comprises oral compositions containing a stain and tartar removing effective amount of a strontium EDTA complex substantially free of strontium ions from sources other than the strontium EDTA complex and an anti-caries effective amount of inorganic water soluble ionic fluoride salt as a source of soluble fluoride. Typical such ionic fluorides include ammonium fluoride and alkali metal, alkaline earth metal and heavy metal fluoride salts, for example, sodium fluoride, potassium fluoride, lithium fluoride, stannous fluoride, stannic fluoride, barium fluoride, sodium fluorsilicate, ammonium fluorosilicate, sodium fluorozirconate, sodium monofluorophosphate and the like ionic fluoride salts generally acceptable in oral formulations as a source of soluble fluoride. Ammonium fluoride, alkali metal and tin fluorides, particularly sodium and stannous fluorides, respectively, and sodium monofluorophosphate are preferred.

The most preferred source of fluoride in the dentifrice compositions of this invention is a water soluble alkali metal monofluorophosphate salt such as sodium monofluorophosphate, lithium monofluorophosphate and potassium monofluorophosphate, preferably sodium monofluorophosphate. In addition, other monofluorophosphate salts which have sufficient water solubility for use in the dentifrices of the instant invention include those described in U.S. Pat. No. 4,046,872 issued September 6, 1977 to Robert Mitchell et al, for example, ammonium monofluorophosphate, magnesium monofluorophosphate and aluminum monofluorophosphate. In addition, the term "monofluorophosphate" includes water soluble monofluoropolyphosphates such as $Na_4P_3O_9F$, $K_4P_3O_9F$, $(NH_4)_4P_3O_9F$,

$Na_3KP_3O_9F$, $(NH_4)_3NaP_3O_9F$ and $Li_4P_3O_9F$.

The fluoride component will generally be present in the dentifrice compositions in an amount of from about 0.1 to about 5.0 weight percent based on the total weight of the composition and preferably in an amount of from about 0.5 to about 1% and most preferably in an amount of from about 0.75 to about 0.85% by weight.

Accordingly, the oral compositions of this invention comprise an oral vehicle, a stain and tartar removing effective amount of a strontium EDTA complex substantially free of strontium ion from sources other than the strontium EDTA complex and an effective anti-caries amount of a soluble ionic fluoride salt.

As used herein, the term "oral composition" means a product which in the ordinary course of usage is not intentionally swallowed for purposes of systemic administration of the particular therapeutically active ingredient(s), but is retained in the oral cavity for a time sufficient to contact substantially all of the dental surfaces and/or oral tissues for purposes of local activity. Typical oral compositions for purposes of the present invention are those containing at least an amount of water sufficient to solubilize strontium and fluoride components. The preferred oral compositions are liquid dentifrices such as mouthwashes, rinses, sprays and the like, whether in the form of a concentrate to be diluted prior to use or in final ready-to-use form, and semi-solid dentifrices such as dental creams, pastes and gels, all of which compositions contain substantial amounts of water, generally far more than enough to maintain strontium and fluoride ions in solution.

The present invention thus provides, as a preferred embodiment, a water-containing oral composition comprising an effective stain and tartar removing amount of strontium EDTA complex substantially free of strontium ions from sources other than the strontium EDTA complex, an effective anti-caries amount of a water soluble inorganic fluoride salt, and an oral vehicle containing at least sufficient water to solubilize, at least in part, said strontium EDTA complex and said fluoride salt.

It is understood, however, that it is within the broader aspect of the invention to include other oral compositions with oral vehicles such as lozenges, chewing gums and the like wherein water may be present during their manufacture but little if any when finished. In certain sugarless gums, for example, there can be used as the binder ingredient a solution of sorbitol in water containing from about 10% to about 80%, preferably from about 50% to about 75% by weight of the sorbitol in water. In others, there is used a gum acacia-in-water system containing from about 30% to about 60%, preferably from about 45% to about 50%, by weight of gum acacia powder in water.
Similarly, lozenge formulations may contain water
in aqueous systems during formation of the lozenge
mass.   Incorporation of the strontium EDTA complex
and ionic fluoride into such formulations may readily
be accomplished without fear of precipitating
insoluble fluorides by simply utilizing an appropriately prepared aqueous solution of the strontium
EDTA complex and fluoride components.   After incorporation, the drying of the oral composition, as in
the case of lozenges, is then performed to remove
all or part of the moisture.

As an additional novel aspect of this invention,
therefore, there is provided an aqueous solution
which comprises water and, dissolved therein, an
effective stain and tartar removing amount of a
strontium EDTA complex substantially free of strontium ion from sources other than the strontium EDTA
complex, preferably in an amount sufficient to provide from about 0.1 to about 25 weight percent of
theoretical strontium element and an effective anti-
caries amount of a water soluble inorganic ionic
fluoride salt, preferably in an amount sufficient
to provide from about 25 to about 15000 ppm of
fluoride ion.   Also provided is a dilute ethanolic
solution consisting of water, from about 5 to about
40 percent by weight of ethanol, and said amounts
of strontium element and fluoride ion, such solution providing a stable mouthwash base to which
optional ingredients may be added.

The heretofore mentioned oral formulations are exemplary only. Many additional water-containing oral formulations are described in the prior art and, in carrying out this invention, such formulations can be employed so long as there is at least sufficient water to solubilize some of both the strontium and fluoride components either in the final product or during the course of their manufacture.

The subject oral compositions are generally prepared in accordance with art-recognized practices. In general, any conventional ingredients can be used so long as they are compatible, that is, do not interfere with the activity of the strontium EDTA complex or the fluoride component.

As noted previously, the most preferred oral composition is in the form of a liquid dentifrice. Mouthwashes, rinses, sprays, etc. generally comprise a water or water/alcohol solvent system and optionally other ingredients such as flavor, sweeteners, humectants and the like. The alcohol, generally from about 5 to about 40 percent, by weight, helps solubilize the flavoring oils and provides an antibacterial effect although other antibacterial or antiseptic agents can also be incorporated in minor amounts, generally at levels from about 0.01 to about 2.0 percent by weight.

The pH of the liquid compositions of this invention should be from acidic pH 3.0 to pH 8.5 preferably within the range of from about 3.0 to about 7.0, and most preferably from about 5.5 to 6.5. A pH higher than 8.5 should be avoided since the stability of the composition is thereby affected. If desired, the acidic pH of a particular liquid composition if deemed too low can be adjusted to a higher pH up to 8.5 by addition of suitable alkali, for example, a dilute solution of ammonium, sodium or potassium hydroxide.

Since the problem of strontium/fluoride incompatibility is obviated by the present invention, the subject compositions can be prepared without undue concern for the order or manner of incorporating the two essential components. For example, the preferred liquid dentifrices can be conveniently prepared by simple addition of ingredients in no particular order to the water or water/alcohol solvent system containing the strontium EDTA complex and fluoride salts, such other optional ingredients being, for example, a humectant such as glycerin, sorbitol, polyethylene glycol and the like to give a moist feel in the mouth, generally up to about 20.0 percent by weight, and preferably from about 5.0 to about 20.0 percent by weight; a natural or synthetic sweetening agent such as dextrose, levulose, saccharin, cyclamate and the like, generally from about 0.05 to about 2.0 percent by weight; a flavoring agent such as peppermint oil, spearmint oil, clove oil, anise oil, orange oil, wintergreen oil (methyl salicylate) and the like, generally from about 0.01 to about 2.0 percent by weight; and a surface-active or sudsing agent such as, for example, a sodium alkylbenzene sulfonate, a sodium alkyl sulfate or a nonionic or anionic organic synthetic detergent, generally from about 0.05 to about 10.0 percent by weight and preferably from about 0.5 to about 5.0 percent by weight, all of which are conventional surfactants utilized in dentifrice formulations.  Cationic surface active ingredients are not favored since they may give rise to incompatibility with fluoride ions.

The preferred surfactants are sodium lauryl sulfate, particularly for toothpastes, and the nonionic type of synthetic detergents, particularly for mouthwashes.  The nonionic synthetic detergents which can be used herein may be broadly defined as compounds produced by the condensation of a hydrophilic alkylene oxide group with an organic hydrophobic compound which may be aliphatic or alkylaromatic in nature.  The length of the hydrophilic or polyoxyalkylene radical which is condensed with any particular hydrophobic group can be readily adjusted to yield a water-soluble compound having the desired degree of balance between hydrophilic and hydrophobic elements.

For example, a well known class of nonionic

synthetic detergents is commercially available/under
by Wyandotte Chemicals Co.(USA)

the trade name of "Pluronic®".  These compounds are formed by condensing ethylene oxide with a hydrophobic base formed by the condensation of propylene oxide
with propylene glycol.

Other suitable nonionic synthetic detergents
include: the polyethylene oxide condensates of alkyl
phenols, those derived from the condensation of
ethylene oxide with the product resulting from the
reaction of propylene oxide and ethylene diamine,
the condensation product of aliphatic alcohols having
from 8 to 18 carbon atoms, in either straight chain
or branched chain configuration, with ethylene oxide,
and the polyoxyethylene derivatives of fatty acid
partial esters of sorbitol anhydride and commercially
available/under the trade name "Tween"® by Atlas Chemical Industries Inc. (USA)

In the substantially solid or semi-solid oral
compositions of this invention, such as dental
creams, pastes and gels, the liquids and solids
should be proportioned to form an extrudable creamy
mass of desirable consistency. In general, liquids
in these formulations will comprise chiefly water,
glycerin, sorbitol, propylene glycol or the like,
including suitable mixtures thereof. It is advantageous usually to use a mixture of both water and
a humectant or binder such a glycerin or sorbitol,
preferably glycerin. The total liquid content will
generally be about 20 to 75 percent by weight of
the formulation. It is also preferred to use a
gelling agent such as a natural or synthetic gum and gum-like material, for example, Irish moss, gum traganth, xanthan gum, Veegum regular, sodium carboxymethylcellulose, polyvinylpyrrolidone, starch and the like. The Irish moss and sodium carboxymethylcellulose are compatible particularly and are preferred gelling agents. The gum content is usually in an amount up to about 10 percent and preferably about 0.5 to 5 percent by weight of the formulation.

An essential ingredient in dental cream formulations is an effective abrasive amount of a suitable dental abrasive, generally from about 10 to about 60 percent by weight and preferably from about 20 to about 50 percent by weight. As noted previously, this abrasive must not interact with either the strontium or fluoride component. Typical compatible abrasives include, for example, insoluble metaphosphates, finely divided silicas, bentonite and the like. The preferred abrasive is silica.

Various other materials may be incorporated as adjuvants in dental creams. Examples thereof are coloring or whitening agents, preservatives, silicones, chlorophyll compounds, ammoniated materials such as urea, diammoniumphosphate and mixtures thereof, and other constituents. A small amount of colloidal silica, for example, is often incorporated into toothpaste formulations as a thickener, giving some body to the formulation upon swelling when in contact with water. The foregoing adjuvants are suitably selected and incorporated in the instant compositions in amounts which do not substantially adversely affect the properties and characteristics desired for the particular type of composition.

For some purposes it may be desirable to include antibacterial agents in the dental creams. Typical antibacterial agents which may be used in amounts of about 0.01 percent to about 5 percent, preferably about 0.05 percent to about 1.0 percent by weight of the composition include: $N^1$-(4-chloro-benzyl)-$N^5$(2,4-dichlorobenzyl)biguanide, p-chlorophenyl biguanide, 4-chlorobenzyhydryl biguanide, 4-chlorobenzhydrylguanylurea, N-3-lauroxpropyl-$N^5$-p-chlorobenzylbiguanide, 1,6-di-p-chlorophenylbi-guanidohexane, 1-(lauryldimethylammonium)-8-(p-chloro-benzyldimethylammonium)octane dichloride, 5,6-dichloro-2-guanidinobenzimidazole, $N^1$-p-chlorophenyl-$N^5$-lauryl-biguanide, 5-amino-1,3-bis(2-ethylenyl)-5-methylhexahydropyrimidine, and their non-toxic acid addition salts.

Tooth desensitization agents such as, for example, a nitrate of potassium, lithium or sodium disclosed in U.S.A. Patent 3,863,006 issued January 28, 1975 to Milton Hodosh, may also be incorporated in tooth desensitizing amounts, generally up to about 20% and preferably about 5% by weight.

Any suitable flavoring or sweetening materials may also be employed. Examples of suitable flavoring constituents include the flavoring oils, e.g.

oils of spearmint, peppermint, wintergreen, sassafras, clove, sage, eucalyptus, marjoram, cinnamon,
lemon and orange, as well as sodium methylsalicylate. Suitable natural and synthetic sweetening
agents include sucrose, lactose, maltose, sorbitol,
sodium cyclamate, ammonium glycyrrhizinate and its
derivatives and saccharin. Suitably, flavor and
sweetening agents may together comprise from about
0.01 to 5 percent or more by weight of the dental
cream.

The dental cream should have a pH practicable
for use. An acidic to neutral pH from about 3.0 to
about 8.5 is preferred with 4.0 to 6.5 most preferred.
An appropriate acidic buffer may be used to stabilize
the pH, for example, an acid/salt buffer such as
citric acid/citrate, malic acid/malate, adipic acid/
adipate and the like. The pH determination is made
on a 10% aqueous suspension of the dentifrice. If
necessary, conventional acidic materials may be
added to adjust the pH as desired.

When visually clear gels are employed, polishing agents comprising alkali metal aluminosilicate
complexes are particularly useful, since they have
refractive indices close to the refractive indices
of gelling agent-liquid (including water and/or
humectant) systems commonly used in dentifrices.
In clear gels where the refractive index is an
important consideration, about 3-30% by weight of
water, 0 to 80% by weight of glycerin, and about

0079611

20-80% by weight of sorbitol is preferably employed.

A gelling agent, such as a natural or synthetic gum or gum-like material, typically Irish moss, sodium carboxymethylcellulose, methyl cellulose, hydroxyethylcellulose, gum tragacanth, polyvinylpyrrolidone, starch, or preferably hydroxypropyl methylcellulose or the Carbopols® by B.F. Goodrich Chemical Co., USA (for example, Carbopol 934, 940 and 941) or the like is usually present in toothpastes in an amount up to about 10% by weight, preferably in the range of from about 0.5 to about 5%.

In a toothpaste or gel, the liquids and solids are proportioned to form a creamy or gelled mass which is extrudable from a pressurized container or from a collapsible, for example, aluminum or lead, tube.

As noted previously, aqueous solutions of the strontium and fluoride components may be utilized in formulating other oral compositions wherein little or no moisture is present in the final product. For example, a chewing gum suitable for use as an oral vehicle herein comprises a gum base and flavoring materials such as those mentioned above for dentifrices. The flavoring materials are present at a level of 0.01% to about 2.0% of the final chewing gum composition. The gum base is a chewable plastic gum material such as natural rubber, chicle, polyvinyl acetate, ester gum, coumarone resin, and paraffin wax. The gum base is typically made from a mixture of two or more plastic gum materials to achieve a preferred degree of plasticity for chewing. Corn syrup is generally added as a softener and binder for the chewing gum and sugar is optionally added as a filler and sweetener. A typical chewing gum suitable as a carrier herein comprises 15% to 30% gum base, 15% to 20% corn materials. An aqueous solution of the strontium and fluoride components may be incorporated into the corn syrup prior to admixture with the gum base.

Lozenges suitable as carriers herein comprise a hard sugar candy base and one or more flavoring materials. The flavoring materials are present at levels between 0.01 to 2.0%. Optionally, lozenges can contain various other materials. A typical lozenge suitable as an oral vehicle in this invention is a hard candy comprised of a hard candy base containing 0.05% to 1.5% flavor. The hard candy base is a solidified solution of amorphous sugar which is generally formed from a sugar solution which has been cooked at high temperature so as to remove nearly all of the moisture. The flavoring materials and the aqueous strontium/fluoride solution are added before the moisture is removed. The flavoring materials mentioned hereinbefore for dentifrices are also exemplary of those suitable for use in lozenges.

The following are exemplary oral compositions of this invention, it being understood that the strontium EDTA Complex in the formulation is substantially free of strontium ions from sources other than the strontium EDTA Complex. That is, the strontium EDTA complex can be for example, that produced according to the aforementioned U.S. Patent 4,224,310.

### Example 1 - Mouthwash

| Component | Amount |
|---|---|
| Strontium EDTA complex | 5.0 g |
| Sodium fluoride | 0.5 g |
| Alcohol U.S.P. | 52.6 ml |
| Glycerin | 20.0 g |
| Pluronic® F-127 | 10.0 g |
| Cinnamaldehyde | 0.9 ml |
| Tween® 80 | 1.0 g |
| Saccharin insoluble | 0.5 g |
| Menthol-L-natural | 0.38 g |
| Clove oil | 0.14 ml |
| Color (FD&C* Red 40 + D&C* Red 33) | 0.003 g |
| Distilled water, to make | 1000.0 ml |

The Pluronic® F-127, Tween® 80, saccharin, menthol, cinnamaldehyde and clove oil are dissolved in the alcohol. The strontium EDTA complex is dissolved in about 750 ml distilled water with vigorous stirring (magnetic stirrer) and the sodium fluoride added followed by the glycerin and color. To this aqueous solution is added the alcoholic solution and the final product made to volume with distilled water. The mouthwash is clear and stable and no visible precipitation occurs over normal shelf-life.

Remarks: *: FD & C and D & C Colorants are artificial colors permissible for use in food, drugs, or cosmetics by the FDA (USA)

### Example 2 - Toothpaste

| Component | % w/w |
| --- | --- |
| Strontium EDTA complex | 10.0 |
| Sodium monofluorophosphate | 0.76 |
| Xanthan gum | 0.55 |
| Glycerin | 8.0 |
| Sorbitol solution (70% w/w) | 42.0 |
| Silica | 32.0 |
| Sodium saccharin | 0.45 |
| Sodium benzoate | 0.1 |
| Sodium lauryl sulfate | 2.0 |
| Colloidal silica | 1.0 |
| Flavor | 1.5 |
| Color (FD&C Blue #1) | 0.003 |
| Distilled water, to make | 100.00 |

The xanthan gum and sodium saccharin are mixed and dispersed in the glycerin. The sorbitol is mixed with about an equal volume of water and the glycerin mixture added to it. The silica, sodium lauryl sulfate and colloidal silica are mixed in at high speed under vacuum and the flavoring and color added. The strontium EDTA complex and sodium monofluorophosphate are dissolved in the remaining water (heated slightly). The aqueous solution is stable and clear with no precipitation of insolubles. The sodium benzoate is added to the aqueous solution and the latter admixed with the glycerin mixture. The formulation provides a theoretical value of about 2.07 percent by weight of strontium element and about 1000 ppm of soluble fluoride.

## Example 3 - Chewing Gum

| Component | Weight % |
|---|---|
| Strontium EDTA complex | 3.0 |
| Stannous fluoride | 0.10 |
| Distilled water | 2.50 |
| Gum base | 26.00 |
| Sucrose | 52.00 |
| Corn syrup | 17.00 |
| Flavor | 1.90 |

The strontium EDTA complex and stannous fluoride are dissolved in the water (heated slightly) and the aqueous solution admixed with the corn syrup prior to incorporation with the other components.

## Example 4 - Lozenge

| Component | Weight % |
|---|---|
| Strontium EDTA complex | 6.5 |
| Ammonium fluoride | 0.10 |
| Sugar | 80.00 |
| Corn syrup | 11.00 |
| Flavor oil | 0.65 |
| Color | 0.25 |
| Tragacanth mucilage | 5.00 |
| Distilled water | 2.00 |

The strontium EDTA complex and ammonium fluoride are dissolved in the water (heated slightly) and the aqueous solution admixed with the corn syrup prior to incorporation with the other ingredients into a tenacious lozenge mass with sufficient plasticity for molding and cutting into desired shapes and sizes prior to drying.

## Example 5 - Mouthwash

| Component | % w/w |
|---|---|
| Strontium EDTA complex | 7.5 |
| Sodium fluoride | 0.5 |
| Alcohol U.S.P. | 15.0 |
| Glycerin | 10.0 |
| Flavor | 0.2 |
| Saccharin | 0.03 |
| FD&C Color | 0.3 |
| Distilled water, to make | 100 ml |

The strontium EDTA complex, sodium fluoride and alcohol are dissolved in about 50 ml water. The other ingredients are then added to this ethanolic solution.

That strontium EDTA complex can be unexpectedly incorporated into oral compositions under the proper environment without unacceptable loss of available soluble fluoride is shown by reference to the following comparative formulations and the data obtained when these compositions were subjected to comparative testing.

| Ingredients | Formulation No. — wt. % of Ingredient | | | |
|---|---|---|---|---|
| | 1 | 2 | 3 | 4 |
| Disodium EDTA·$2H_2O$ | 20 | 20 | 4.41 | 4.41 |
| Strontium chloride·$6H_2O$ | 20 | -- | -- | -- |
| Strontium carbonate | -- | 7.92 | 1.75 | -- |
| Strontium hydroxide | -- | -- | -- | 3.13 |
| Silica | 19 | 19 | 19 | 19 |
| Sylox® 24 (silica gel) | 6 | 6 | 6 | 6 |
| Tween® 80 | 0.5 | 0.5 | 0.5 | 0.5 |
| Xanthan gum (Keltrol® F) | 1 | 1 | 1 | 1 |
| Flavoring | 1 | 1 | 1 | 1 |
| Propylene glycol | 25 | 25 | 25 | 25 |
| Alcohol U.S.P. | 0.9 | 1.0 | 1.0 | 1.0 |
| Sodium monofluorophosphate | 0.76* | 0.76* | 0.76* | 0.76* |
| Distilled water | 5.84 | 17.82 | 39.58 | 38.2 |

*Equals 1000 ppm (theoretical) soluble fluoride.

Formulation 1 represents a composition formed according to the disclosure of Example 2 of previously mentioned Schole et al U.S. Patent 3,699,221, modified to replace strontium carbonate abrasive with a non-interfering silica/silica gel abrasive system and containing 22.75% by weight strontium EDTA complex. In Formulation 2, which is otherwise similar to Formulation 1, the 22.75% strontium EDTA complex is formed according to the method disclosed in the aforementioned Shah U.S. Patent 4,224,310 so that the complex is free of strontium ion from sources other than the complex. Formulation 3 is identical to Formulation 2 except that the level of strontium EDTA complex present in the formulation is 5% by weight. Formulation 4 is identical to Formulation 3 except that the 5% by weight strontium EDTA complex is formed by the reaction similar to that disclosed in said U.S. Patent 4,224,310 but where strontium hydroxide is employed as the reactant in place of strontium carbonate.

Formulations 1 through 4 were aged at room temperature, 37°C and 45°C for up to one month and the amount of total soluble fluoride available is determined periodically in parts per million (ppm) by standard measurements. The results are indicated in the table below. Each formulation was formulated to initially contain at least a theoretical 1000 ppm of total soluble fluoride.

| Formulation No. | PPM Soluble Fluoride | | | |
|---|---|---|---|---|
| | 1 | 2 | 3 | 4 |
| Initial* | 1000 | 1000 | 1000 | 1000 |
| Room Temp./2 weeks | 730 | ** | ** | ** |
| Room Temp./1 month | 630 | 880 | 900 | 910 |
| 37° C/1 month | 570 | 800 | 860 | 910 |
| 45° C/2 weeks | 660,760 | 710 | 860 | 870 |
| 45° C/1 month | 580 | 680 | 820 | 900 |

*Formula amount
**Indicates no measurement taken

As can be seen by reference to the data obtained, an unacceptable loss of available soluble fluoride occurs when strontium EDTA complex prepared as disclosed by Schole et al is employed (Formulation 1) whereas acceptable available soluble fluoride is retained when strontium EDTA complex substantially free of strontium ion from sources other than the strontium EDTA complex is employed (Formulations 2, 3 and 4).

## CLAIMS

1. An oral composition comprising an oral vehicle, a tartar and stain removing effective amount of a strontium EDTA complex substantially free of strontium ions from sources other than the strontium EDTA complex and an effective anti-caries amount of a water soluble inorganic ionic fluoride salt.

2. The composition of claim 1 wherein said fluoride salt is a member selected from the group consisting of ammonium fluoride, sodium monofluorophosphate, an alkali metal fluoride, and a tin fluoride.

3. The composition of any of claims 1 and 2 which is a water-containing oral composition.

4. The composition of claim 3 wherein said composition is a mouthwash.

5. The composition of claim 3 wherein said composition is a toothpaste.

6. An aqueous solution comprising water and, dissolved therein, a tartar and stain removing effective amount of a strontium EDTA complex substantially free of strontium ions from sources other than the strontium EDTA complex and an effective anti-caries amount of a water soluble inorganic ionic fluoride salt.

7. An aqueous, ethanolic solution comprising from about 5 to about 40% by weight of ethanol in water and, dissolved therein, a tartar and stain removing effective amount of a strontium EDTA complex substantially free from strontium ions from sources other than the strontium EDTA complex and an effective anti-caries amount of a water soluble inorganic ionic fluoride salt.

8. The composition of any of claims 6 and 7 wherein said fluoride salt is a member selected from the group consisting of ammonium fluoride, sodium monofluorophosphate, an alkali metal fluoride, and a tin fluoride.

9. Use of the compositions of any of claims 6, 7 and 8 in oral compositions, particularly in dentifrices.